# FASCICULE DE BREVET EUROPEEN

(11) **EP 4 507 708 B1**
(45) Date de publication et mention de la délivrance du brevet: **15.10.2025**
(21) Numéro de dépôt: 23723076.8
(22) Date de dépôt: 11.04.2023
(51) Int. Cl.: A61K 38/00, A61P 25/28, C07K 7/06

(54) **PEPTIDE UTILE POUR LE TRANSPORT DE MOLÉCULES AU TRAVERS DE BARRIÉRES CELLULAIRES**
PEPTID ZUM TRANSPORT VON MOLEKÜLEN DURCH ZELLBARRIEREN
PEPTIDE SUITABLE FOR TRANSPORTING MOLECULES ACROSS CELL BARRIERS

(30) Priorité: 11.04.2022 FR 2203309
(43) Date de publication de la demande: 19.02.2025
(73) Titulaire: Université Grenoble Alpes, 38400 Saint-Martin-d'Hères (FR); Institut National de la Santé et de la Recherche Médicale, 75013 Paris (FR)
(72) Inventeur: LOMBARDI, Charlotte, 26300 Rochfort Samson (FR); MOULIN, Marcelle, 74000 Annecy (FR); GHEZZI, Catherine, 38000 Grenoble (FR)
(74) Mandataire: Germain Maureau
(86) Numéro de dépôt international: PCT/FR2023/050519
(87) Numéro de publication internationale: WO 2023/198985

(56) Documents cités:
- WO-A1-2018/078140
- SWAPNA BERA ET AL, BIOCHEMISTRY, vol. 55, no. 35, 24 August 2016 (2016-08-24), pages 4982 - 4996, XP055691861, ISSN: 0006-2960, DOI: 10.1021/acs.biochem.6b00518
- CHOI-FONG CHO ET AL: "Blood-brain-barrier spheroids as an in vitro screening platform for brain-penetrating agents", NATURE COMMUNICATIONS, vol. 8, no. 1, 6 June 2017 (2017-06-06), XP055692204, DOI: 10.1038/ncomms15623
- KARDANI KIMIA ET AL: "Cell penetrating peptides: the potent multi-cargo intracellular carriers", EXPERT OPINION ON DRUG DELIVERY, vol. 16, no. 11, 2 November 2019 (2019-11-02), GB, pages 1227 - 1258, XP055776584, ISSN: 1742-5247, DOI: 10.1080/17425247.2019.1676720

## Description

### Domaine technique

La présente invention concerne un nouveau peptide et son utilisation comme vecteur pour le transport de molécules d'intérêt après leur fusion audit peptide formant ainsi une protéine de fusion (ci-après dénommé peptide conjugué ou conjugué peptidique) au travers des barrières cellulaires, en particulier la barrière hémato-encéphanique (BHE) pour le diagnostic, le pronostic ou le traitement de pathologies du système nerveux central (SNC), par exemple les accidents vasculaires cérébraux (AVC), les tauopathies, plus particulièrement la maladie d'Alzheimer ou la maladie de Parkinson, etc....

Dans la description ci-dessous, les références entre crochets ([ ]) renvoient à la liste des références à la fin du texte.

### Etat de la technique

Les barrières du cerveau protègent notre système nerveux central (composé du cerveau et de la colonne vertébrale) en contrôlant l'oxygène, les nutriments et en bloquant l'entrée des substances ou des micro-organismes dommageables, pour le cerveau. Il existe 3 barrières entre le sang et le système nerveux central (SNC) : la barrière hémato-encéphalique (BHE), la barrière sang-liquide cérébrospinal ou plexus choroïdes (CP) et la barrière arachnoïde qui sert d'interface entre le cerveau et le liquide céphalorachidien (Abbott et al., Neurobiol. Dis., 37 : 13-25, 2010) [1].

La BHE concentre la plupart des efforts de franchissement puisque c'est l'interface majeure entre le sang et le SNC. Or la BHE est le principal obstacle au développement de nouvelles molécules diagnostiques ou thérapeutiques ciblant le cerveau. En effet seulement 2% des molécules développées la franchissent (Pardridge, Molecular Interventions, 3(2) : 90-105, 2003) [2] et ce sont des molécules de petites tailles (<600 Da) (Pardridge, Expert Opinion on Drug delivery, DOI : 10.1517/17425247.2016.1171315, 2006) [3].

La BHE se compose de petits capillaires propres au cerveau, entourés par les cellules endothéliales formant l'endothélium, élément clé de cette barrière.

La surface totale de cet endothélium représente 12 à 20 m² soit des kilomètres d'interface entre les capillaires et le cerveau (De Boer et Gaillard, Clin. Pharmacokinet., 46(7) : 553-576, 2007) [4]. Mais le franchissement de cette interface est très contrôlé et sélectif. En effet, les cellules endothéliales sont étroitement accolées par des jonctions serrées, formant ainsi une véritable forteresse entre les capillaires et le cerveau. Les jonctions serrées sont constituées de protéines d'adhésion capables de sélectionner les molécules pouvant franchir ou non cet endothélium. On parle alors de transport paracellulaire qui s'oppose au transport transcellulaire où le passage se fait par traversée de la membrane des cellules endothéliales (Patel et Patel, CNS Drugs, DOI : 10.1007//s40263-016-0405-9, 2017) [5].

Pour ce deuxième type de transport, la traversée de l'endothélium peut se faire par diffusion passive pour les petites molécules non polaires, les liquides et les alcools ou via un transporteur présent à la surface des cellules comme pour le glucose ou les acides aminés. Mais lorsque la taille des molécules est trop importante pour l'utilisation d'un transporteur, le passage de la membrane se fait alors par transcytose. Certaines protéines comme l'insuline ou la transferrine se lient à des récepteurs présents sur la membrane des cellules endothéliales et qui médient la transcytose. Pour les autres dépourvues de récepteurs, la transcytose ne peut se faire que si ces molécules remplissent les conditions nécessaires à leur adsorption telle que la présence de patch de charges positives à leur surface (Patel et Patel, 2017) [5].

Une fois cet endothélium traversé, les molécules se heurtent, dans une moindre mesure, à d'autres types de cellules constituant la BHE. En effet, les cellules endothéliales sont partiellement entourées par les péricytes, cellules contractiles participant à la structure de l'endothélium et des capillaires et jouant un rôle dans la vasodilatation ou la vasoconstriction de ces derniers. La communication entre les capillaires, les péricytes et les cellules endothéliales se fait grâce à la membrane basale. N'étant pas une couche complètement jointe, les molécules s'en affranchissent plus facilement.

Enfin, les astrocytes qui jouent un rôle de médiateurs entre les capillaires et les neurones, qui participent à la formation des jonctions serrées avec les péricytes et qui aident au maintien de la structure de la BHE et des capillaires constituent le dernier obstacle. Mais tout comme les péricytes, leur assemblage n'est pas continu et leur passage n'est donc probablement pas aussi complexe que celui de l'endothélium.

La recherche de nouvelles molécules ciblant une pathologie du SNC doit donc allier la capacité de franchissement de la BHE à l'activité biologique, la bio-distribution, la faible toxicité et la stabilité de celle-ci. Peu de molécules y parviennent car la recherche se focalise quasi systématiquement sur la recherche d'une cible et d'un ligand adaptés à la problématique de façon déconnectée du franchissement de la BHE. Pour franchir cette BHE, plusieurs stratégies sont envisagées. Elles se divisent en 2 catégories, les stratégies invasives et non invasives (Dong, Theranostics, 8(6) : 1481-1493, 2018) [6]. Parmi les stratégies non invasives prometteuses, on trouve l'utilisation de molécules « chevaux de Troie » utilisant les récepteurs à la surface de la BHE ou l'utilisation de peptides de pénétration cellulaire (CPP) adsorbées par transcytose (Zhou et al., WIREs Nanomed Nanobiotechnol., 13 : e1695, 2021) [7].

Les peptides, lorsqu'ils sont combinés à des molécules, y compris les molécules de grande taille telles que les protéines, sont capables d'augmenter leur pénétration cérébrale en augmentant leur capacité de passage au travers de la BHE. Plusieurs peptides ont été développés pour le franchissement de la BHE, linéaires ou cycliques, entre 5 et 50 acides aminés. Certains utilisent les récepteurs présents au niveau de la BHE tandis que d'autres utilisent la diffusion passive. Ces derniers, appelés peptides de pénétration cellulaire (CPP), sont généralement amphipatiques et/ou cationiques et ne sont pas spécifiques de la BHE. Ils augmentent néanmoins le passage des molécules au travers des membranes cellulaires et, combinés à d'autres stratégies pour les acheminer préférentiellement à la BHE, peuvent être déterminants pour la délivrance des molécules à cible cérébrale (Oller-Salvia, Chem. Soc. Rev., 45(17) : 4690-4707, 2016) [8].

Il demeure donc un grand besoin d'identifier de nouveaux médicaments pour traiter les pathologies du SNC telles que par exemple les accidents vasculaires cérébraux (AVC), les tauopathies par exemple la maladie d'Alzheimer ou la maladie de Parkinson. Les molécules à visée thérapeutique en cours de développement pour le cerveau échouent pour la plupart à atteindre leur cible cérébrale en raison de leur incapacité à traverser la BHE. De plus près d'un milliard de personnes sont atteintes d'un trouble neurologique (hors troubles psychiatriques). De nombreuses molécules efficaces *in vitro* ont été trouvées pour ces pathologies mais ont besoin d'un vecteur pour leur permettre d'atteindre leur cible. Le marché de la délivrance de molécules thérapeutiques au travers de la BHE atteindra 7,4 milliards de dollars US en 2028 d'après Emergen research en 2021.

### Description de l'invention

Dans le but de faire franchir la BHE à des biomolécules pour le diagnostic ou la thérapie de pathologies cérébrales, les inventeurs ont conçu par bio-informatique un nouveau CPP (ci-après dénommé peptide TB) avec une structure tridimensionnelle formant une hélice alpha stable, de petite taille, avec des charges positives réparties à la surface et un point isoélectrique théorique supérieur à 10 (12,70 d'après l'outil en ligne Protparam d'expasy) - ces critères étant essentiels pour le franchissement de la BHE, la stabilité du peptide TB et des protéines d'intérêt auquel il est attaché.

Ce peptide TB de séquence en acides aminés RQRIWFQNRRRSRKIKK (SEQ ID NO: 1), codée par la séquence en nucléotides CGCCAGCGCATTTGGTTTCAGAACCGCCGCCGCAGCCGCAAAATTAAA AAA (SEQ ID NO : 2), a démontré sa capacité à transporter au travers de la BHE une molécule d'intérêt, à savoir le nanocorps 2C5 (VHH, PM=10-15 kDa) de séquence en acides aminés QVQLVQSGGGLVQAGGSLRLSCAASGRTFSSDTLAWFRQAPGKEREFVA SISPSGGVTYYEDSVKGRFTISRDNSKNTVLLQMNSLTPEDTAVYYCNRDP KYGNTRYWGQGTQVTVSSAAA (SEQ ID NO : 3) capable de détecter les formes pathologiques (forme oligomère) de la protéine Tau (Demande de Brevet FR 3058143) [9], après fusion.

Le conjugué peptidique 2C5-TB résultant a la séquence en acides aminés MQVQLVQSGGGLVQAGGSLRLSCAASGRTFSSDTLAWFRQAPGKEREFV ASISPSGGVTYYEDSVKGRFTISRDNSKNTVLLQMNSLTPEDTAVYYCNRD PKYGNTRYWGQGTQVTVSSAAA**RQRIWFQNRRRSRKIKK**HHHHHH* (SEQ ID NO: 4), codée par la séquence en nucléotides ATGCAGGTGCAGCTGGTGCAGTCTGGGGGAGGATTGGTGCAGGCTGG GGGTTCTCTGAGACTCTCCTGTGCAGCCTCTGGACGCACCTTCAGTAG CGATACCCTGGCGTGGTTCCGCCAGGCGCCAGGGAAGGAGCGTGAGT TTGTAGCGTCTATTAGTCCCAGTGGTGGTGTCACATACTATGAAGACTC CGTGAAGGGCCGATTCACCATCTCCAGAGACAACAGCAAGAACACGGT GTTGCTGCAAATGAACAGCCTGACACCTGAGGACACGGCCGTCTATTA CTGTAACCGGGACCCCAAGTACGGTAACACTAGATACTGGGGCCAGGG GACCCAGGTCACCGTCTCCTCAGCGGCCGCA**CGCCAGCGCATTTGGTTT CAGAACCGCCGCCGCAGCCGCAAAATTAAAAAA***CATCACCACCATCA CCAT*TAA (SEQ ID NO : 5), où la séquence du nanocorps 2C5 est celle soulignée, la séquence du peptide TB est celle en gras et la séquence en italique est une étiquette polyhistidine comprenant au moins 6 résidus histidine. Il est à noter que 3 alanines sont ajoutées après la séquence de 2C5 se terminant par TVSS et avant la séquence du peptide Tb commençant par RQR, pour servir de linker. Toutefois ce linker n'est pas indispensable au bon transport de la molécule d'intérêt au travers de la BHE (données non représentées).

Ce peptide TB de séquence en acides aminés RQRIWFQNRRRSRKIKK (SEQ ID NO: 1), codée par la séquence en nucléotides CGCCAGCGCATTTGGTTTCAGAACCGCCGCCGCAGCCGCAAAATTAAA AAA (SEQ ID NO : 2), a également démontré sa capacité à transporter au travers de la BHE une seconde molécule d'intérêt, à savoir la neurotensine (NT), de séquence en acides aminés RRPYIL (SEQ ID NO : 6), qui est un neuropeptide provoquant une hypothermie lorsqu'il est présent dans le cerveau mais qui est incapable seul de traverser la BHE.En conséquence, le conjugué peptidique TB-NT résultant, correspondant à la séquence en acides aminés **RQRIWFQNRRRSRKIKK**RRPYIL (SEQ ID NO : 7), où la séquence de la neurotensine (NT) est celle soulignée et la séquence du peptide TB est celle en gras.

Ce peptide TB représente donc un vecteur efficace capable de transporter au travers des barrières cellulaires, en particulier de la BHE, des molécules d'intérêt à visée diagnostique, pronostique et thérapeutique de pathologies du SNC qui échouent pour la plupart à atteindre leur cible cérébrale en raison de leur incapacité à traverser seules la BHE, comme dans le cas du nanocorps 2C5 et de la neurotensine (NT).

La présente invention a donc pour objet un peptide comprenant ou constitué d'un peptide de séquence en acides aminés SEQ ID NO 1.

Ledit peptide peut être synthétisé par tous procédés connus de l'Homme du métier, par exemple à l'aide d'un synthétiseur peptidique ou par synthèse de gène, insertion de celui-ci dans un vecteur d'expression et expression en système bactérien, cellulaire ou acellulaire.

La présente invention a également pour objet un conjugué peptidique comprenant ou constitué d'un peptide selon l'invention, où ledit peptide est lié de manière covalente à une molécule d'intérêt, directement ou via un linker. Selon un mode de réalisation particulier de la présente invention, ledit linker, lorsqu'il est présent, est choisi dans le groupe constitué des peptides, polymères ou agents chimiques permettant le cross-linking. De préférence ledit linker, lorsqu'il est présent, est composé de 1 à 6 acides aminés, de préférence de 3 acides aminés, préférentiellement de 3 alanines.

Selon un mode de réalisation particulier de la présente invention, la molécule d'intérêt est choisie dans le groupe constitué de toute molécule chimique ou biologique d'intérêt diagnostique, pronostique ou thérapeutique. Par exemple, il peut s'agir d'un anticorps (*e.g.* un nanocorps), d'un ADN, d'un ARN, d'un peptide (*e.g.* antalgique, anesthésique, *e.g.* neurotensine), d'un médicament (*e.g.* inhibiteurs de BACE1 (beta-site APP cleaving enzyme 1) ou de Gamma-sécrétases). De préférence, le nanocorps est un nanocorps anti-Tau capable de lier les formes pathologiques précoces de la protéine Tau humaine tout en étant dépourvu de capacité de franchissement de la BHE, de préférence un nanocorps Tau de séquence en acides aminés SEQ ID NO : 3.

Selon un mode de réalisation particulier de la présente invention, le conjugué peptidique comprend ou est constitué de la séquence SEQ ID NO : 4.

Selon l'invention, la molécule d'intérêt liée au peptide TB peut être un neuropeptide (*e.g.* antalgique, anesthésique, *e.g.* neurotensine). De préférence, ledit neuropeptide est une neurotensine capable de provoquer une hypothermie lorsqu'il est présent dans le cerveau tout en étant dépourvu de capacité de franchissement de la BHE, de préférence une neurotensine (NT) de séquence en acides aminés SEQ ID NO : 6.

Selon un mode de réalisation particulier de la présente invention, le conjugué peptidique comprend ou est constitué de la séquence SEQ ID NO : 7.

La présente invention a également pour objet un conjugué peptidique selon la présente invention pour une utilisation comme médicament. En particulier, le conjugué peptidique est utile dans le diagnostic, le pronostic, ou le traitement d'une pathologie du système nerveux central (SNC).

Selon un mode de réalisation particulier de la présente invention, la pathologie du SNC est choisie dans le groupe constitué des tumeurs cérébrales, des accidents vasculaires cérébraux (AVC), de la sclérose en plaques, de la maladie d'Huntington et des tauopathies. Par exemple, la tauopathie est choisie dans le groupe constitué de la maladie de Parkinson, la maladie d'Alzheimer, la maladie de Pick.

La présente invention a également pour objet une séquence en acides nucléique codant pour un peptide ou un conjugué peptidique selon la présente invention, de préférence la séquence en acides nucléiques comprend ou est constitué de la séquence SEQ ID NO : 2 ou de la séquence SEQ ID NO : 5, respectivement.

La présente invention a également pour objet un vecteur d'expression comprenant une séquence en acides nucléiques selon la présente invention, par exemple le plasmide pET15b.

### BREVE DESCRIPTION DES FIGURES

[Figure 1] représente la structure tridimensionnelle du peptide TB.
[Figure 2] représente la confirmation de l'hélice alpha du peptide TB par les programmes PepFold et heliquest.
[Figure 3] représente les conjugués comprenant le peptide TB ou BIP fusionné au nanocorps 2C5.
[Figure 4] représente le modèle cellulaire de la BHE.
[Figure 5] représente l'effet des nanocorps et conjugués (2C5, 2C5-TB et 2C5-BIP) sur la perméabilité au saccharose des cellules endothéliales.
[Figure 6] représente les courbes de clairance du saccharose au travers des cellules endothéliales exposées aux nanocorps et conjugués (2C5, 2C5-TB et 2C5-BIP).
[Figure 7] représente le coefficient de perméabilité en µL/h/cm² des nanocorps et conjugués (2C5, 2C5-TB et 2C5-BIP).
[Figure 8] représente les données des peptides HoxA-13 et pénétratine obtenues par le programme heliquest.
[Figure 9] représente les courbes variations de température corporelles de souris après leur injection en intraveineuse des différents peptides (NT, TB, TAT-NT et TB-NT).

### EXEMPLES

### EXEMPLE 1 : CONCEPTION D'UN NOUVEAU CPP : LE PEPTIDE TB

### Séquence protéique du peptide TB

La séquence du peptide TB a été modélisée en 3D à l'aide du programme COOT pour former une hélice alpha stable avec de nombreuses charges positives réparties à la surface, avec un point isoélectrique (pl) théorique proche de 13 (12,70 d'après protparam) afin d'abaisser ultérieurement le pl des molécules d'intérêt qui lui sont fusionnées.

Le peptide TB a ensuite été synthétisé par synthèse de gène et produit en système bactérien ou par synthèse peptidique à l'aide d'un synthétiseur.

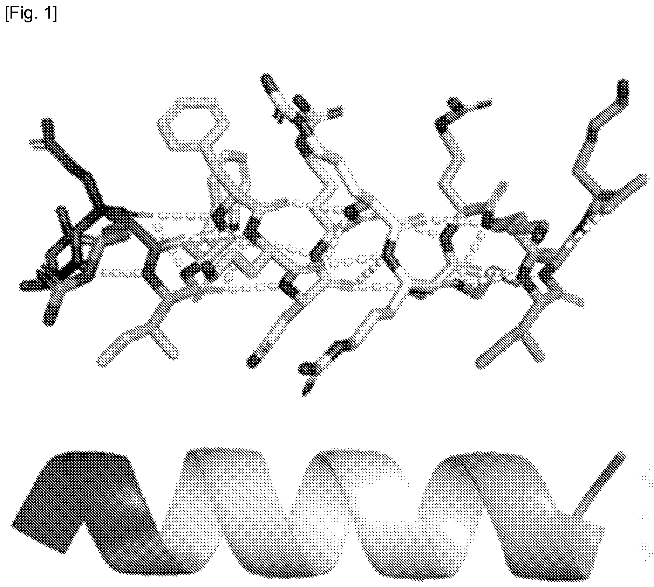

### Structure tridimensionnelle résultante (Fig. 1)

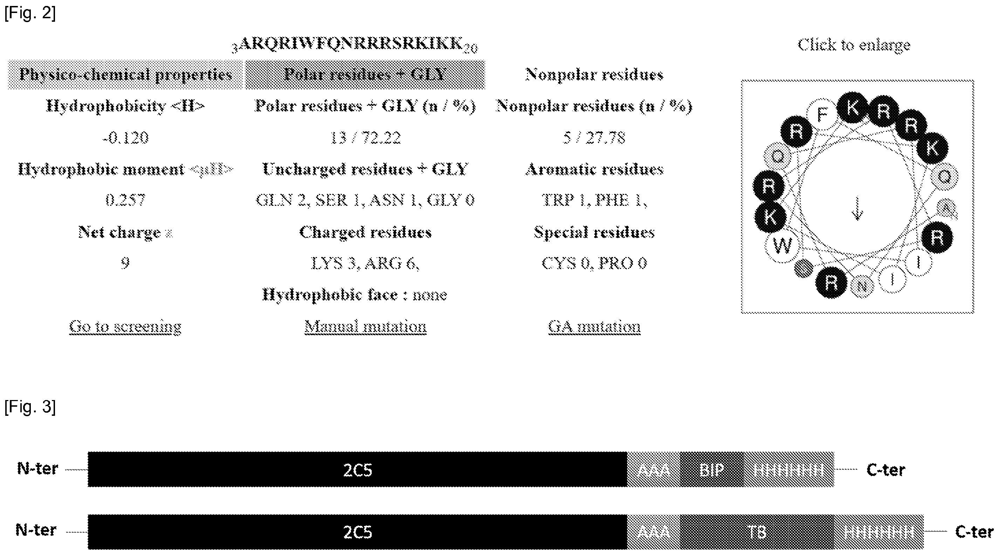

L'hélice alpha du peptide TB a été confirmée par les programmes PepFold et heliquest (Fig. 2).

Le peptide TB a été conçu *in silico* de sorte à avoir des charges positives mieux répartis à sa surface, une meilleure solubilité dans les solvants aqueux et une hélice-α plus stable d'un point de vue structural. L'ensemble de ces propriétés permet au peptide TB, ainsi qu'à tous ses conjugués, de présenter une solubilité accrue dans les solvants aqueux, pour une utilisation *in vivo.* In fine, TB et ses différents conjugués présentent une capacité de franchissement de la BHE plus importante que pour tout autre CPP connu.

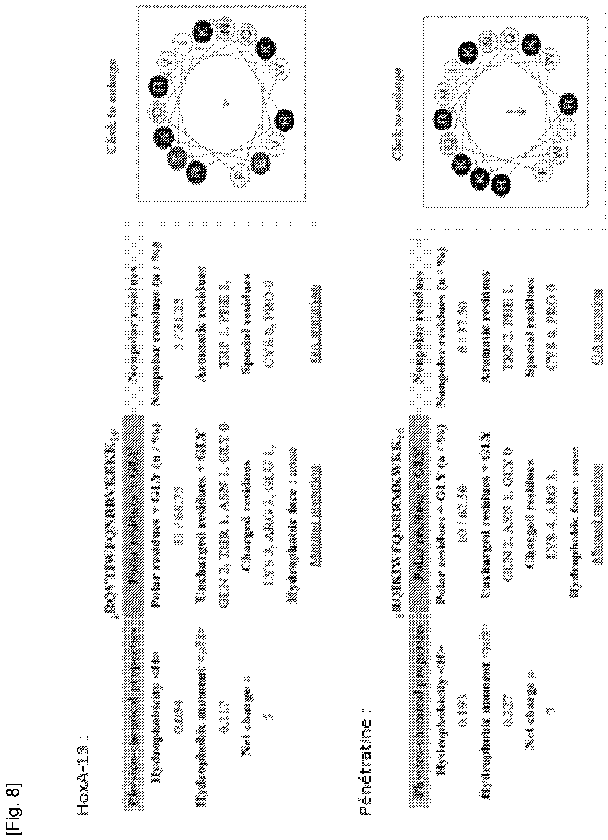

En ce sens, en comparant les propriétés du peptide TB à celles de deux autres CPP connus, le peptide HoxA-13 et la pénétratine, on constate que le peptide TB présente une hydrophobicité négative contrairement à HoxA-13 et à la pénétratine (Fig. 8). Cette différence confère au peptide TB une solubilité dans les solvants aqueux, contrairement à HoxA-13 et la pénétratine, lui permettant de franchir la BHE, notamment lorsqu'il est lié à une molécule d'intérêt, telle que le nanocorps (2C5) ou la neurotensine (NT).

### EXEMPLE 2 : FUSION DU PEPTIDE TB A UN NANOCORPS

### Fusion du peptide TB à un nanocorps ne passant pas la BHE

Pour évaluer la capacité de diffusion au travers d'un modèle de BHE cellulaire différencié, le peptide TB a été fusionné à un nanocorps (2C5) ne passant pas ce modèle mais dont la cible est cérébrale (Demande internationale WO 2018/078140) [11].

La conjugaison du peptide TB au nanocorps 2C5 a été réalisée par fusion de gènes de telle sorte que la protéine résultante se compose, du N-terminus vers le C-terminus, du nanocorps 2C5, d'un linker composé de 3 alanines, du peptide TB et d'une étiquette de 6 histidines. Une fusion similaire avec un CCP prometteur d'après Cho, 2017 [9], le peptide BIP, a également été réalisée (Fig. 3).

Les gènes correspondants ont été insérés dans le plasmide pET15b à l'aide des enzymes de restriction Ncol/BamHI (données non représentées).

### Production des nanocorps 2C5, et des conjugués peptidiques 2C5-TB et 2C5-BIP

Les plasmides codant pour les 3 protéines (2C5, 2C5-TB et 2C5-BIP) ont été insérés dans des bactéries *E*. *co*li Shuffle (Biolabs) selon le protocole du fournisseur.

A partir d'une colonie issue de la transformation, une préculture a été réalisée dans un volume de 25 mL de LB contenant 100 µg/mL d'ampicilline. Cette préculture a été incubée sur la nuit à 37°C avec une agitation de 190 rpm.

Le jour suivant une culture de 1 L a été réalisée en diluant les 25 mL de préculture dans du LB contenant 100 µg/mL d'ampicilline. La préculture a été incubée à 37°C avec une agitation de 190 rpm et lorsque la densité optique mesurée à 600 nm a atteint 0,8, 1 mM d'IPTG a été ajouté pour induire le promoteur T7 du plasmide pET15b. La culture a été incubée pendant 3 heures supplémentaires dans les mêmes conditions.

Les bactéries ont été collectées à l'issue des 3 heures d'induction, par centrifugation 40 min à 9500 G. Le surnageant a été éliminé et le culot bactérien a été resuspendu dans 40 mL de Tampon de lyse (50 mM Tris HCl pH9 pour 2C5-TB et pH8 pour 2C5 et 2C5-BIP, 250 mMNaCl, 30 mM imidazole, 1 mg/ml de lysozyme, 2 pastilles d'antiprotéase complete EDTA free (Roche) et 1 µL de benzonase). La suspension a été mise à 4°C pendant 1 heure sous agitation douce pour permettre la lyse sous l'action du lysozyme. Une étape supplémentaire de sonication a ensuite été réalisée et la fraction soluble contenant les nanocorps ou conjugués a été collectée par centrifugation durant 40 min. à 9500 G.

Les nanocorps possédant une queue poly-histidine, les nanocorps et conjugués ont été purifiés sur une résine d'affinité Ni-NTA agarose (Qiagen). La fraction soluble a été chargée dans une colonne de gravité contenant 1 mL de résine Ni-NTA prééquilibrée en TpA (50 mM Tris HCl pH9 pour 2C5-TB et pH8 pour 2C5 et 2C5-BIP, 250 mM NaCl, 30 mM Imidazole). Puis la résine a été lavée à l'aide de 150 mL de TpA et les nanocorps et conjugués ont été élués par addition de 250 mM d'imidazole dans le TpA.

Des fractions de 1 mL ont été collectées et celles contenant les nanocorps ou conjugués après analyse sur gel SDS 18% ont été réunies et injectées sur une colonne superdex 75 10/300 pour une seconde étape de purification sur un système FPLC Biorad NGC, en tampon PBS.

Les fractions contenant les nanocorps ou conjugués, pures à plus de 95%, ont été réunies et collectées à 1 mg/ml pour les nanocorps 2C5 et les conjugués 2C5-BIP et à 0,8 mg/mL pour les conjugués 2C5-TB.

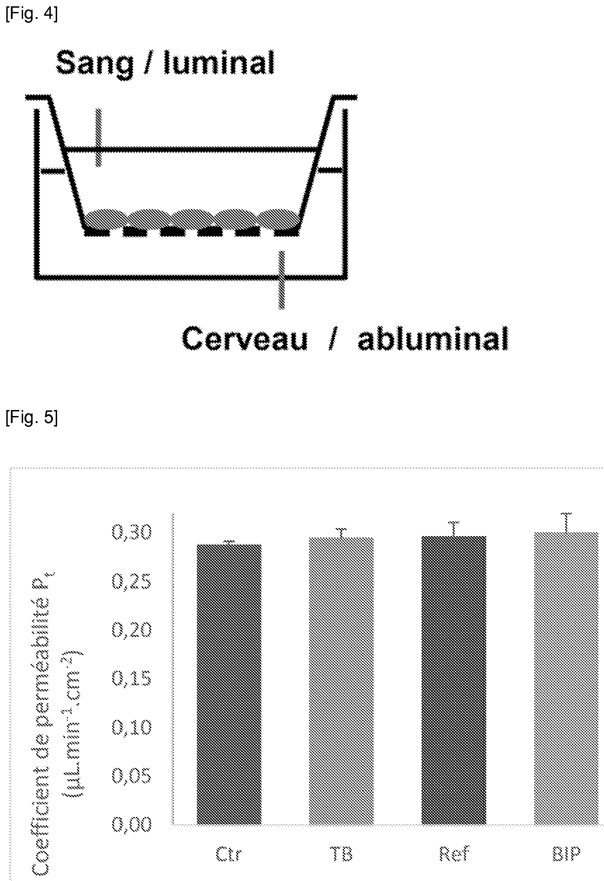

### Evaluation du passage des nanocorps et conjugués peptidiques au travers d'un modèle de BHE cellulaire différencié (Fig. 4)

Des cellules endothéliales de micro-vaisseaux cérébraux préparées à partir de rats mâles âgés de 5 semaines ont été ensemencées sur filtres microporeux afin de :
- évaluer la toxicité des nanocorps et conjugués au cours de leur transfert par la mesure cinétique du transfert de saccharose.
- évaluer la perméabilité des nanocorps et conjugués dans le sens sang/cerveau.

### Conditions expérimentales :

- Les cellules endothéliales ont été ensemencées sur 28 filtres Transwell Polycarbonate (porosité 0,4 µm et diamètre 12 mm), et cultivées en milieu de différenciation.
- La mesure de toxicité (étude A) ou de perméabilité (étude B) des nanocorps et conjugués a été réalisée en milieu HBSS Ca/Mg supplémenté par 0,1 % BSA. Les volumes de transfert ont été respectivement de 280 et 1200 µl pour le compartiment luminal donneur et le compartiment basolatéral accepteur.
- Les nanocorps et conjugués ont été dilués dans le même tampon HBSS Ca/Mg - 0,1 % BSA.
- Le transfert a été initié en ajoutant les nanocorps ou conjugués dans le compartiment supérieur et en transférant le filtre dans un puits contenant HBSS Ca/Mg - 0,1 % BSA.
- L'intégrité des monocouches contrôles ou exposés aux nanocorps ou conjugués (étude A) a été évaluée par la mesure de la perméabilité au saccharose au travers d'une analyse cinétique sur 2 heures (comparable au temps utilisé pour estimer la perméabilité des nanocorps et conjugués).
- Les milieux luminaux et abluminaux collectés pour l'étude B ont été analysés par test ELISA.
- Les groupes sont les suivants :

**[Tableau 1]**

| Etude | A. Toxicité des nanocorps et conjugués | B. Perméabilité des nanocorps et conjugués |
|---|---|---|
| Groupes/nbre de fibres | | |
| Prélèvement luminal vol/temps | 50 µl/120 min | 20 µl/8 min |
| | | 250 µl/120 min |
| Prélèvement abluminal vol/temps | 900 µl/15, 30, 45, 60, 90, 120 min | 250 µl/60 min |
| | | vol total/120 min |

Pour l'étude B, le milieu prélevé à 60 min en basolatéral a été remplacé par le même volume de milieu frais.

### Résultats de l'étude A :

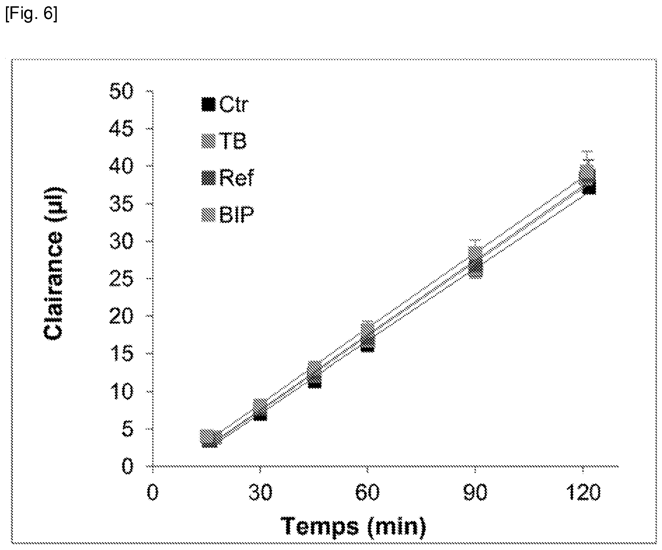

La perméabilité au sucrose des cellules endothéliales reflétant l'intégrité des jonctions serrées n'a pas été modifiée en présence des nanocorps et conjugués (Fig. 5). Les courbes de clairance moyennées pour les 4 filtres ont été linéaires pour les quatre groupes de filtres (Fig. 6).

Ces résultats ont démontré que les trois nanocorps et conjugués (2C5, 2C5-TB et 2C5-BIP) testés n'ont pas d'effet délétère aigu sur la perméabilité des cellules endothéliales au saccharose. Ils valident par conséquent les éventuelles différences observées au sein des valeurs des coefficients de perméabilité pour les 3 nanocorps et conjugués testés.

### Résultats de l'étude B :

Les échantillons collectés à 60 et 120 min ont été analysés par test ELISA pour évaluer la quantité de nanocorps et conjugués qui est passée au cours de l'étude B. Pour pouvoir analyser les différents échantillons par ELISA, il a fallu préalablement les purifier pour ôter la BSA limitant l'adhésion aux plaques 96 puits du test ELISA. Grâce à l'étiquette polyhistidine présente sur les 3 nanocorps et conjugués, des purifications sur plaque 96 puits recouverte de Nickel-NTA ont été réalisées.

Les purifications ont été réalisées comme suit :
- Incubation 1 heure à 4°C des échantillons sur la plaque Ni-NTA.
- Elimination des fractions « non retenues » par pipetage.
- 3 lavages de 200 µL en tampon PBS + 30 mM imidazole.
- Incubation 30 min avec 200 µL de solution d'élution PBS + 300 mM imidazole.
- Transfert des élutions sur plaques 96 puits pour les tests ELISA.

En parallèle, des gammes de concentration des différents nanocorps et conjugués ont été réalisées et mises à incuber sur les mêmes plaques 96 puits que les élutions précédentes, sur la nuit à 4°C. Le jour suivant les fractions non adsorbées ont été éliminées par retournement et un blocage a été réalisé avec une solution de PBS + 1 % BSA durant 1 heure à 23°C. Une solution de PBS + 1 % BSA contenant des anticorps anti-histidine et couplés HRP est venue remplacer la solution de blocage et une incubation de 1 heure à 23°C a été réalisée pour permettre aux anticorps de se lier aux nanocorps et conjugués présents. 3 lavages en PBS + 0,1 % tween ont ensuite été réalisés pour éliminer les éléments non retenus et une solution de TMB a été ajoutée pour révéler par colorimétrie les tests ELISA.

Une lecture d'absorbance a été réalisée à 450 nm après arrêt de la réaction colorimétrique à l'aide d'une solution d'HCl 1N.

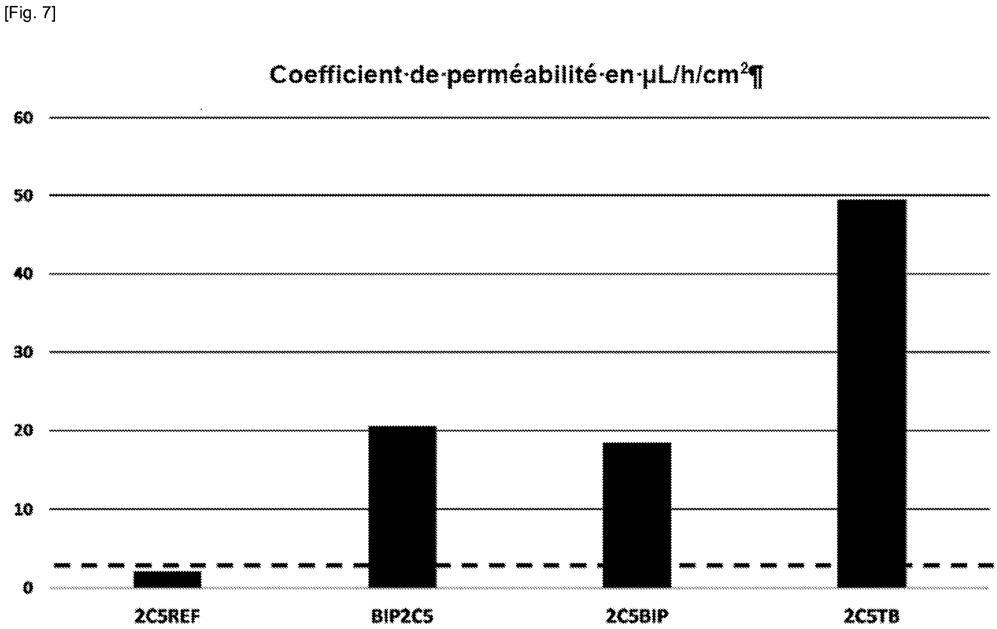

Les gammes de concentration réalisées ont permis de tracer les courbes d'absorbance en fonction de la concentration en nanocorps et conjugués et de déterminer la limite de linéarité ainsi que le coefficient permettant de passer des absorbances aux concentrations dans les échantillons prélevés au cours de l'étude B. Pour pouvoir comparer les quantités de nanocorps et conjugués passées, les concentrations des puits ont ensuite été converties en quantités de nanocorps et conjugué passées par heure et par cm² de modèle. Afin de comparer ces valeurs aux références (un dextran de 15000 Da et le saccharose), la diffusion des nanocorps a été finalement exprimée en coefficient de perméabilité soit en µL/h/cm² (Fig. 7).

Ces résultats ont démontré la capacité du conjugué 2C5-TB à passer au travers de la BHE. Ainsi le peptide TB a démontré sa capacité de vecteur à transporter au travers de la BHE une molécule d'intérêt ne passant pas la BHE lorsqu'administrée seule.

En outre, des études comparatives, dans lesquelles le nanocorps 2C5 a été fusionné à HoxA-13 et à la pénétratine, ont permis de confirmer la capacité unique du peptide TB à être un vecteur de transport au travers de la BHE, d'une molécule d'intérêt telle que le nanocorps 2C5. En effet, les conjugués peptidiques 2C5-pénétratine et 2C5-HoxA-13 n'ont pas pu être solubilisés entièrement lors de l'étape de lyse des bactéries servant à sa production (méthode identique à celle du conjugué 2C5-TB), révélant ainsi une incapacité (ou tout au moins une très faible capacité) à traverser la BHE. Dès lors, ces études ont confirmé que parmi ces trois peptides de pénétration cellulaire (CPP), seul le peptide TB de l'invention permet, lorsqu'il est conjugué au nanocorps 2C5, le transport de cette molécule d'intérêt au travers de la BHE.

### EXEMPLE 3 : FUSION DU PEPTIDE TB à une neurotensine

### Fusion du peptide TB à un neuropeptide ne passant pas la BHE

### Séquences des différents peptides utilisés

NT : RRPYIL
TB : RQRIWFQNRRRSRKIKK
TAT-NT : GRKKRRQRRRPQRRPYIL
TB-NT : RQRIWFQNRRRSRKIKKRRPYIL

### Méthode

La neurotensine (NT) est un neuropeptide qui provoque une hypothermie lorsqu'il est présent dans le cerveau mais qui est incapable d'atteindre seul le cerveau depuis la périphérie. Ce peptide, s'il est fusionné à TB, peut ainsi servir de rapporteur du franchissement de la BHE. Pour cela, les peptides TB, NT et TB-NT ont été synthétisés à l'aide d'un synthétiseur peptidique et la dose de 2,5 mmol/kg a été injectée en intraveineuse chez des souris (n=4 à 6). Un suivi de la température corporelle a été réalisé à l'aide de capsules Anipill^{®} implantés préalablement en intrapéritonéale.

Ces données ont été étudiées comparativement à celles réalisées avec le peptide de pénétration cellulaire de référence TAT, lui aussi fusionné à la neurotensine NT. Le conjugué résultant TAT-NT a été utilisé en contrôle positif, afin de comparer la capacité de TB à faire franchir la BHE à NT, au regard de celle du peptide TAT. Le peptide TAT, originaire du virus de l'immunodéficience humaine (VIH), a été montré en 1999 comme capable de transporter un conjugué au travers de la BHE [10].

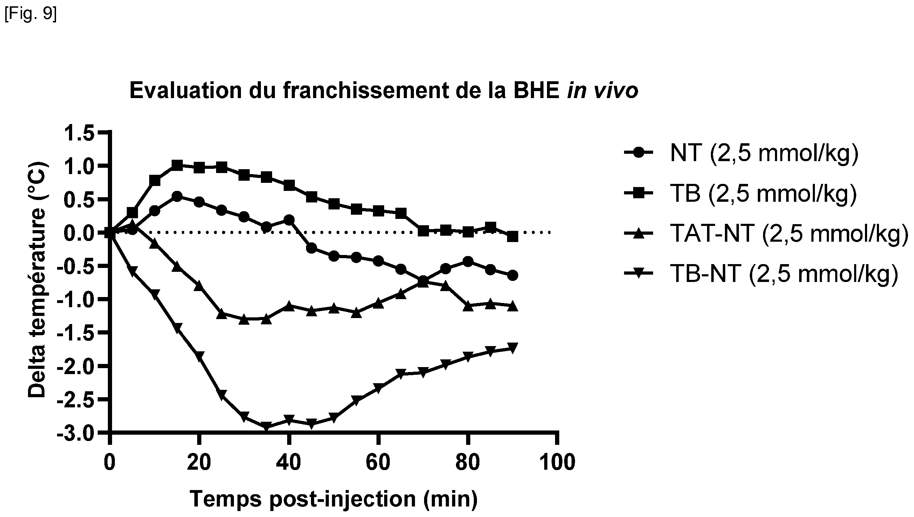

Le graphique résultant de cette étude (Fig.9) montre les variations de température corporelles des souris après leur injection en intraveineuse des différents peptides. Le suivi a été réalisé sur 90 minutes.

### Résultats

Les souris ayant reçu NT seul ne montrent pas de variation de la température dans les minutes qui suivent l'injection et confirme l'absence de passage du sang au cerveau de ce peptide seul. Autrement dit, cette courbe confirme l'incapacité de NT à franchir seule la BHE.

Les souris ayant reçu TB seul ne montrent aucun signe d'hypothermie.

Les souris ayant reçu NT fusionné à TAT montrent une hypothermie d'environ 1 degré Celsius, observée après 30 minutes suivant l'injection. Cela confirme la capacité du peptide TAT à transporter un conjugué au travers de la BHE.

Néanmoins, les souris ayant reçu NT fusionné à TB montrent une hypothermie plus importante d'environ 3 degrés Celsius, observée après 35 minutes suivant l'injection. Ainsi, cela traduit une capacité du peptide TB à faire franchir la BHE à une molécule d'intérêt telle que la neurotensine qui est nettement supérieure à celle du peptide TAT.

Tous les animaux ont retrouvé une température corporelle similaire après 2heures et 30 minutes.

### Liste des références

1. Abbott et al., Neurobiol. Dis., 37 : 13-25, 2010
2. Pardridge, Molecular Interventions, 3(2) : 90-105, 2003
3. Pardridge, Expert Opinion on Drug delivery, DOI : 10.1517/17425247.2016.1171315, 2006
4. De Boer et Gaillard, Clin. Pharmacokinet., 46(7) : 553-576, 2007
5. Patel et Patel, CNS Drugs, DOI : 10.1007//s40263-016-0405-9, 2017
6. Dong, Theranostics, 8(6) : 1481-1493, 2018
7. Zhou et al., WIREs Nanomed Nanobiotechnol., 13 : e1695, 2021
8. Oller-Salvia, Chem. Soc. Rev., 45(17) : 4690-4707, 2016
9. Demande de Brevet FR 3058143
10. Schwarze et al., 1999

## Revendications

1. Peptide comprenant un peptide de séquence en acides aminés SEQ ID NO 1.

2. Conjugué peptidique comprenant le peptide selon la revendication 1, où ledit peptide est lié de manière covalente à une molécule d'intérêt, directement ou via un linker.

3. Conjugué peptidique selon la revendication 2, où le linker est composé de 1 à 6 acides aminés, de préférence de 3 alanines.

4. Conjugué peptidique selon la revendication 2 ou 3, où la molécule d'intérêt est choisie dans le groupe constitué d'un anticorps, d'un ADN, d'un ARN, d'un peptide, et d'un médicament.

5. Conjugué peptidique selon la revendication 4, où la molécule d'intérêt est un nanocorps, de préférence un nanocorps anti-Tau.

6. Conjugué peptidique selon la revendication 5, où le nanocorps anti-Tau comprend la séquence en acides aminés SEQ ID NO : 3.

7. Conjugué peptidique selon la revendication 6 comprenant la séquence en acides aminés SEQ ID NO : 4.

8. Conjugué peptidique selon l'une quelconque des revendications 2 à 7, pour une utilisation comme médicament.

9. Conjugué peptidique selon l'une quelconque des revendications 2 à 7, pour une utilisation dans le diagnostic, le pronostic ou le traitement d'une pathologie du système nerveux central (SNC).

10. Conjugué peptidique pour une utilisation selon la revendication 9, où la pathologie du SNC est choisie dans le groupe constitué des tumeurs cérébrales, des accidents vasculaires cérébraux (AVC), de la maladie de Huntington, de la sclérose en plaques et des tauopathies.

11. Conjugué peptidique pour une utilisation selon la revendication 10, où la tauopathie est choisie dans le groupe constitué de la maladie de Parkinson, de la maladie de Pick, et de la maladie d'Alzheimer.

12. Séquence d'acides nucléiques codant pour un peptide selon la revendication 1 ou un conjugué peptidique selon l'une quelconque des revendications 2 à 7, de préférence une séquence en acides nucléiques comprenant la séquence SEQ ID NO : 2 ou la séquence SEQ ID NO : 5.

13. Vecteur d'expression comprenant une séquence en acides nucléiques telle que définie dans la revendication 12.

## Patentansprüche

1. Peptid, umfassend ein Aminosäuresequenzpeptid SEQ ID NO: 1.

2. Peptidkonjugat, umfassend das Peptid nach Anspruch 1, wobei das Peptid direkt oder über einen Linker kovalent mit einem Molekül von Interesse gebunden ist.

3. Peptidkonjugat nach Anspruch 2, wobei der Linker aus 1 bis 6 Aminosäuren, vorzugsweise 3 Alaninen, besteht.

4. Peptidkonjugat nach Anspruch 2 oder 3, wobei das Molekül von Interesse aus der Gruppe ausgewählt wird, die aus einem Antikörper, einer DNA, einer RNA, einem Peptid und einem Arzneimittel besteht.

5. Peptidkonjugat nach Anspruch 4, wobei das Molekül von Interesse ein Nanokörper, vorzugsweise ein Anti-Tau-Nanokörper, ist.

6. Peptidkonjugat nach Anspruch 5, der Anti-Tau-Nanokörper umfassend die Aminosäuresequenz SEQ ID NO: 3.

7. Peptidkonjugat nach Anspruch 6, umfassend die Aminosäuresequenz SEQ ID NO: 4.

8. Peptidkonjugat nach einem der Ansprüche 2 bis 7, zur Verwendung als Arzneimittel.

9. Peptidkonjugat nach einem der Ansprüche 2 bis 7 zur Verwendung bei der Diagnose, Prognose oder Behandlung einer Erkrankung des zentralen Nervensystems (ZNS).

10. Peptidkonjugat für die Verwendung nach Anspruch 9, wobei die ZNS-Pathologie aus der Gruppe ausgewählt wird, die aus Hirntumoren, Schlaganfall, Huntington-Krankheit, Multipler Sklerose und Tauopathien besteht.

11. Peptidkonjugat zur Verwendung nach Anspruch 10, wobei die Tauopathie aus der Gruppe ausgewählt wird, die aus Parkinson-Krankheit, Pick-Krankheit und Alzheimer-Krankheit besteht.

12. Nukleinsäuresequenz, kodierend ein Peptid nach Anspruch 1 oder ein Peptidkonjugat nach einem der Ansprüche 2 bis 7, vorzugsweise eine Nukleinsäuresequenz, umfassend die Sequenz SEQ ID NO: 2 oder die Sequenz SEQ ID NO: 5.

13. Expressionsvektor, umfassend eine Nukleinsäuresequenz nach der Definition von Anspruch 12.

## Claims

1. A peptide comprising a peptide of amino acid sequence SEQ ID NO: 1.

2. A peptide conjugate comprising the peptide according to claim 1, wherein said peptide is covalently bonded to a molecule of interest, directly or via a linker.

3. The peptide conjugate according to claim 2, wherein the linker is composed of 1 to 6 amino acids, preferably 3 alanines.

4. The peptide conjugate according to claim 2 or 3, wherein the molecule of interest is selected from the group consisting of an antibody, DNA, RNA, a peptide, and a drug.

5. The peptide conjugate according to claim 4, wherein the molecule of interest is a nanobody, preferably an anti-Tau nanobody.

6. The peptide conjugate according to claim 5, wherein the anti-Tau nanobody comprises the amino acid sequence SEQ ID NO: 3.

7. The peptide conjugate according to claim 6 comprising the amino acid sequence SEQ ID NO: 4.

8. The peptide conjugate according to any one of claims 2 to 7, for use as a drug.

9. The peptide conjugate according to any one of claims 2 to 7, for use in the diagnosis, prognosis, or treatment of a pathology of the central nervous system (CNS).

10. The peptide conjugate for use according to claim 9, wherein the CNS pathology is selected from the group consisting of brain tumors, strokes (CVA), Huntington's disease, multiple sclerosis, and tauopathies.

11. The peptide conjugate for use according to claim 10, wherein the tauopathy is selected from the group consisting of Parkinson's disease, Pick's disease, and Alzheimer's disease.

12. A nucleic acid sequence encoding a peptide according to claim 1 or a peptide conjugate according to any one of claims 2 to 7, preferably a nucleic acid sequence comprising the sequence SEQ ID NO: 2 or the sequence SEQ ID NO: 5.

13. An expression vector comprising a nucleic acid sequence as defined in claim 12.
